Europäisches Patentamt

European Patent Office

Office européen des brevets

(19)

(11) Publication number: **0 080 854**
A1

## (12) EUROPEAN PATENT APPLICATION

(21) Application number: **82306243.5**

(22) Date of filing: **23.11.82**

(51) Int. Cl.³: **A 23 K 1/165**
**A 23 K 1/18**

(30) Priority: **28.11.81 GB 8136006**

(43) Date of publication of application:
**08.06.83 Bulletin 83/23**

(84) Designated Contracting States:
**AT BE CH DE FR GB IT LI LU NL SE**

(71) Applicant: **Pfizer Limited**
**Ramsgate Road**
**Sandwich Kent CT13 9NJ(GB)**

(84) Designated Contracting States:
**GB**

(71) Applicant: **Pfizer Corporation**
**Calle 15 1/2 Avenida Santa Isabel**
**Colon(PA)**

(84) Designated Contracting States:
**BE CH DE FR IT LI LU NL SE AT**

(72) Inventor: **Pimblett, Ian John**
**Hilbre 96 St. George's Road**
**Sandwich Kent(GB)**

(72) Inventor: **Snarey, Michael**
**Pound House St. Mary's Close**
**Eastry Sandwich Kent(GB)**

(74) Representative: **Moore, James William**
**Pfizer Limited Ramsgate Road**
**Sandwich Kent CT13 9NJ(GB)**

(54) **Method of improving growth of poultry and feed compositions therefor.**

(57) A method of improving the efficiency of feed utilisation and/or growth of poultry comprises orally administering thyrotropin releasing hormone (TRH). The invention also provides poultry feed compositions containing TRH.

EP 0 080 854 A1

Croydon Printing Company Ltd.

This invention relates to a method of improving the efficiency of feed utilisation and/or growth of poultry by the oral administration of thyrotropin releasing hormone (TRH).

TRH is a naturally occurring tripeptide identified as pyroglutamylhistidylprolinamide. It is a hypothalamic neurohormone which stimulates the release and perhaps synthesis of thyrotropin from the anterior pituitary gland. TRH has been administered by injection into sheep and cattle to improve feed utilisation and growth, and has also been administered to cows to improve milk production.

We have now surprisingly discovered that TRH is effective in improving the efficiency of feed utilisation and/or growth of poultry when given orally.

Thus the present invention provides a method of improving the efficiency of feed utilisation and/or growth of poultry which comprises orally administering TRH. The invention also provides a poultry feed composition comprising a poultry feed and a feed efficiency and/or growth improving amount of TRH.

In accordance with the present invention, the oral administration of TRH is most conveniently achieved by adding it to the drinking water or feed supplied to the animals. The TRH may be added to a supplementary feed, to the all-mash feed or to only part of the daily feed ration. In practice addition to the normal mixed feed is preferred because of the greater convenience.

- 3 -

Because only very low levels of TRH are needed, care must be taken to ensure even distribution throughout the feed and this is achieved, for example, by adsorbing a solution of TRH onto an inert carrier material, such as cellulose powder, which is then dried and the resulting powder mixed with the feed.

Conventional poultry feeds may be used containing, for example, cereals such as maize, corn, wheat or barley; protein sources such as fish or meat by-products; fats; vitamins and minerals; each in an amount sufficient to meet the nutritional requirements of the animals in accordance with standard veterinary practice.

The TRH is added to the feed to give an amount of between 5 µg and 150 µg per kg of feed, a level of between 10 and 100 µg per kg being especially preferred for improving the efficiency of feed utilisation and growth of chickens. The TRH containing feed is normally provided to the poultry on a free-access basis from shortly after hatching, (e.g. as day-old chicks), until, or until shortly before slaughter, thereby providing continuous administration of TRH throughout the growth of the animals. Naturally, as the birds grow their food intake increases and the amount of TRH taken by any particular animal also rises. Thus, for example in the case of chickens given feeds containing from 5 to 100 µg per kg of TRH, the average daily intake of TRH varies from an approximate level of from 0.1 to 2 µg, in the first few days after birth, up to an approximate level of from 0.5 to 10 µg at four weeks of age.

- 4 -

Other additives, for example antibiotics or coccidiostats, may be included in the feed, if desired, in accordance with standard veterinary practice.

While it is preferred to administer TRH on a continuous basis in the feed as described above, it is also possible to provide the TRH intermittently or at specific periods during the growth of the animals. The TRH may also be administered separately from the feed, for example, as a paste, powder, granules, juice, syrup or concentrate, or it may be added to the drinking water or some other drink e.g. milk. Such preparations are prepared in accordance with acceptable veterinary practice and contain a sufficient amount of TRH to provide each animal with a daily dose of TRH within the ranges specified above.

The method and compositions of the present invention are illustrated by the following examples:

### EXAMPLE 1

A chick feed composition was prepared of the following composition:

|                    | %    |
|--------------------|------|
| Maize              | 20.0 |
| Barley             | 30.0 |
| Wheat              | 12.4 |
| Herring            | 2.0  |
| Meat and Bone Meal | 5.0  |
| Soya flour         | 13.5 |

- 5 -

| Wheatfeed | 15.0 |
|---|---|
| Liquid Fat | 0.6 |
| Salt | 0.2 |
| Limestone | 0.9 |
| Dicalcium Phosphate | 0.3 |
| Vitamin premix | 0.2 |

TRH (0.11 mg) was dissolved in ethanol (100 ml) and this was added to Avicel (Trade Mark, 100 g) to give a slurry which was dried using a rotary evaporator. The resulting powder was thoroughly mixed with 25 kg of the above feed to give a premix which was finally mixed with a further 85 kg of feedstuff to give 110 kg of mash containing 10 µg of TRH per kg of feed.

### Typical Analysis (as fed basis)

| Oil | 3.7 % |
|---|---|
| Protein | 18.5 % |
| Fibre | 4.2 % |
| Energy Content | 11.6 MJ/kg |

Feed compositions are similarly prepared containing TRH in an amount of 5,50 and 100 µg/kg of mixed feed.

### EXAMPLE 2

Poultry feed compositions for broilers were prepared as described in Example 1 having the following compositions:

- 6 -

|  | Starter % | Finisher % |
|---|---|---|
| Maize | 25.0 | 25.0 |
| Wheat | 42.9 | 50.4 |
| Herring | 9.25 | 6.75 |
| Meat and Bone Meal | 7.0 | 6.75 |
| Soya | 11.5 | 7.5 |
| Wheatfeed | 3.5 | 2.75 |
| Liquid Fat | 0.33 | 0.33 |
| Methionine | 0.07 | 0.07 |
| Salt | 0.25 | 0.25 |
| Vitamin Premix | 0.2 | 0.2 |

Typical Analysis (as fed basis)

| Oil (%) | 3.7 | 3.5 |
|---|---|---|
| Protein (%) | 22.2 | 19.2 |
| Fibre (%) | 2.7 | 2.5 |
| Energy Content (MJ/kg) | 12.6 | 12.8 |

TRH was added in an amount of 50, 75 and 100 µg per kg of mixed feed.

EXAMPLE 3

The growth promoting and feed efficiency improving properties of TRH in poultry were shown by the following feeding trial using one-day old broiler chicks.

The chickens were housed in four-tier brooders, each divided into eight pens, two pens per level. Day old, sexed, broiler strain chicks were allocated, usually nine males and nine females to each pen. Treatments and controls were allocated to the pens such that four pens were used for each treatment regime, each treatment being represented in each brooder and on each level across the brooders.

The feed compositions containing various amounts of TRH were prepared as described in Examples 1 or 2 and were provided to the chicks on a free access basis. Records were kept of mortality and of the amount of feed supplied to each pen. At three and four weeks of age all birds were weighed and the weight of all uneaten feed recorded. The live weight gain of the birds was calculated and compared with an untreated control group to give a percentage difference in live weight. Similarly the amount of feed consumed was divided by the live weight of the animals in the group to give a feed conversion ratio (which gives a measure of the amount of feed required to produce 1kg increase in body weight), and this was also compared with the control group and the % difference calculated.

Results obtained in such trials indicate that TRH, when added to the feed at 10 and 50 µg/kg was effective in increasing the growth of the chickens and in improving feed conversion efficiencies.

## CLAIMS

1.  A method of improving the efficiency of feed utilisation and growth of poultry which comprises orally administering TRH.

2.  A method as claimed in claim 1 wherein the TRH is administered by adding to the feed supplied to the poultry.

3   A poultry feed composition comprising a poultry feed and a feed efficiency or growth improving amount of TRH.

4.  A poultry feed composition as claimed in claim 3 containing an amount of from 5 to 150 µg TRH per kg of feed.

5.  A chick feed composition containing from 10 to 100 µg TRH per kg of feed.

CLAIMS FOR AUSTRIA

1. A process for preparing a poultry feed composition which comprises adding TRH to a poultry feed.

2. A process as claimed in claim 1 wherein the TRH is added in an amount of from 5 to 150 µg per kilogram of feed.

3. A process as claimed in claim 1 or claim 2 wherein a solution of TRH is absorbed onto an inert carrier material and the material is dried and mixed with the poultry feed.

4. A process as claimed in claim 3 wherein the inert carrier material is cellulose powder.

0080854

## DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
|---|---|---|---|
| X | DE-A-2 253 274 (ABBOTT LABORATORIES) * Page 2, paragraph 3; claims 1, 4, 5 * | 1,2 | A 23 K 1/165 A 23 K 1/18 |
| A,P | --- Patent Abstracts of Japan, vol. 6, no. 94, 2 June 1982 & JP-A-57-26549 ----- | | |

TECHNICAL FIELDS SEARCHED (Int. Cl. ³)

A 23 K 1/00

The present search report has been drawn up for all claims

| Place of search BERLIN | Date of completion of the search 07-02-1983 | Examiner SCHULTZE D |
|---|---|---|